# FASCICULE DE BREVET EUROPEEN

(11) **EP 3 234 606 B1**
(45) Date de publication et mention de la délivrance du brevet: **24.07.2019**
(21) Numéro de dépôt: 15821140.9
(22) Date de dépôt: 16.12.2015
(51) Int. Cl.: G01N 33/543, C12Q 1/04, B01L 3/00

(54) **PROCEDE ET DISPOSITIF DE DETERMINATION DE LA PRESENCE D'UN MICROORGANISME DANS DES SELLES AVEC PRETRAITEMENT AU CHARBON ACTIF**
VERFAHREN UND VORRICHTUNG ZUR BESTIMMUNG DER PRÄSENZ EINES MIKROORGANISMUS IN STUHL MIT AKTIVKOHLEVORBEHANDLUNG
METHOD AND DEVICE FOR DETERMINING THE PRESENCE OF A MICRO-ORGANISM IN STOOLS WITH ACTIVATED CARBON PRETREATMENT

(30) Priorité: 16.12.2014 FR 1462481
(43) Date de publication de la demande: 25.10.2017
(73) Titulaire: Biomérieux, 69280 Marcy-L'Etoile (FR)
(72) Inventeur: BRIAND, Hélène, 69210 L'arbresle (FR); DURAFFOURG COLLOMB, Anne, 01600 Saint Didier De Formans (FR)
(74) Mandataire: Sarlin, Laure V.
(86) Numéro de dépôt international: PCT/FR2015/053535
(87) Numéro de publication internationale: WO 2016/097596

(56) Documents cités:
- WO-A1-2007/081330
- WO-A2-2005/068647
- CN-A- 103 923 826
- KR-A- 20140 004 457
- US-A1- 2010 024 530
- US-A1- 2012 122 149
- US-B1- 8 232 105
- S. A. CHISHOLM: "Non-invasive diagnosis of Helicobacter pylori infection in adult dyspeptic patients by stool antigen detection: does the rapid immunochromatography test provide a reliable alternative to conventional ELISA kits?", JOURNAL OF MEDICAL MICROBIOLOGY, vol. 53, no. 7, 1 juillet 2004 (2004-07-01), pages 623-627, XP055217914, ISSN: 0022-2615, DOI: 10.1099/jmm.0.05502-0
- SHUVRA KANTI DEY ET AL: "Comparison of immunochromatography, PCR and culture methods for the detection of Campylobacter bacteria", JOURNAL OF MICROBIOLOGICAL METHODS, vol. 91, no. 3, 1 décembre 2012 (2012-12-01), pages 566-568, XP055217894, ISSN: 0167-7012, DOI: 10.1016/j.mimet.2012.09.034
- R. KRAUSSE ET AL: "Evaluation of a Rapid New Stool Antigen Test for Diagnosis of Helicobacter pylori Infection in Adult Patients", JOURNAL OF CLINICAL MICROBIOLOGY, vol. 46, no. 6, 2 avril 2008 (2008-04-02), pages 2062-2065, XP055217897, ISSN: 0095-1137, DOI: 10.1128/JCM.02387-07
- THOMAS REGNATH ET AL: "Accurate detection of Campylobacter spp. antigens by immunochromatography and enzyme immunoassay in routine microbiological laboratory", EUROPEAN JOURNAL OF MICROBIOLOGY AND IMMUNOLOGY, vol. 4, no. 3, 1 septembre 2014 (2014-09-01), pages 156-158, XP055217898, ISSN: 2062-509X, DOI: 10.1556/EUJMI-D-14-00018

## Description

L'invention concerne le domaine technique des immunoessais, et en particulier, des immunoessais adaptés à la détection de pathogènes présents chez un patient, dont la présence peut être mise en évidence à partir des selles dudit patient. Plus précisément, l'invention concerne un procédé de détection d'au moins un antigène d'un microorganisme dans un échantillon liquide de selles ou obtenu à partir de selles d'un patient, ainsi qu'un dispositif de détection par immunochromatographie, adapté à la mise en oeuvre d'un tel procédé.

Les diarrhées représentent à l'échelle mondiale un problème de santé majeure. Dans les pays développés, impact économique des gastro-entérites est important et les dépenses médicales engendrées pour les traiter représentent un coût financier très élevé. Ces infections sont la cause d'une morbidité importante et représentent l'un des motifs majeurs des consultations médicales. Dans les pays défavorisés, les gastro-entérites très fréquentes persistent toute l'année, engendrant une mortalité très élevée. Les populations ne disposant pas d'accès à l'eau potable, sont les plus gravement touchées par cette maladie.

Les agents responsables des gastro-entérites peuvent être des pathogènes bactériens, viraux ou parasitaires. A titre d'exemples de tels pathogènes, on peut citer les microorganismes suivants : *Clostridium difficile, Salmonella, Shigella,* rotavirus, norovirus, adenovirus, *Entamoeba histolytica.*

Le norovirus, particulièrement résistant aux facteurs environnementaux est, de plus en plus souvent, mis en cause au cours d'épisodes diarrhéiques. Ce virus est présent tout autant dans les pays industrialisés que dans les pays en voie de développement et est impliqué dans les épidémies de gastro-entérites touchant toutes les tranches de la population. D'après l'étude bibliographique de Patel MM (Patel MM., et al., Systematic literature review of role of norovirus in sporadic gastroenteritis. Emerg Infect Dis. 2008 Aug; 14(8) :1224-31), le norovirus serait responsable, chez les enfants de moins de cinq ans, vivant dans les pays industrialisés, de 900 000 visites médicales et pas moins de 64 000 cas nécessiteraient une hospitalisation. Dans les pays en voie de développement, un million d'enfants serait touché et le nombre de décès avoisinerait 200 000 morts par an. Chez les enfants hospitalisés de moins de cinq ans, il s'agit du deuxième agent pathogène impliqué dans les gastro-entérites après le rotavirus (Lorrot M., et al., Epidemiology and clinical features of gastroenteritis in hospitalised children: prospective survey during 2-years period in a Parisian hospital, France. Eur J Clin Microbiol Infect Dis. 2011 Mar; 30(3) :361-8*.*).

En cas de signes de gastro-entérites, il est donc nécessaire de disposer de tests qui permettent d'identifier le ou les agents pathogène(s), afin d'apporter rapidement la réponse médicale appropriée. Les agents responsables des gastro-entérites sont, bien souvent, transmis par les fèces.

Les symptômes ne durant que quelques jours, la recherche d'anticorps n'a pas d'intérêt car la réponse immunitaire est trop tardive. Pour réaliser un diagnostic de routine, des techniques immunochromatographiques (également nommées techniques d'immunoessai par flux latéral), basées sur la recherche du virus et/ou d'au moins un analyte représentatif de la présence du virus, dans les prélèvements fécaux sont proposées. Ces techniques sont faciles à mettre en oeuvre, tout en donnant une réponse rapide : le résultat peut être connu en 15 minutes après la mise en oeuvre du protocole d'essai. Une étude réalisée par Ambert-Balay K. et Pothier P. en novembre 2012 présente l'état de l'art des tests immunochromatographiques disponibles sur le marché, dans le cas du norovirus : « Evaluation of 4 immunochromatographic tests for rapid detection of norovirus in faecal samples» J. Clin. Virol 2013 Mar ; 56(3):194-8. Les résultats de sensibilité pour différents tests commerciaux reportés dans cette publication sont repris ci-après :

| | **RIDA© QUICK** | **Immunocard STAT©** | **NOROTOP©** | **SD BIOLINE** |
|---|---|---|---|---|
| **Résultats de l'étude de sensibilité, réponses Génogroupe I+Génogroupe II** | 52% | 35% | 51% | 41% |

Ces résultats montrent que la sensibilité des tests Immunochromatographiques commercialisés pour la recherche du norovirus est faible.

Les selles sont des milieux très complexes qui incluent la présence de bactéries commensales de la flore intestinale, de cellules épithéliales, de résidus de la digestion.... Ceci nécessite, pour tenter d'améliorer la sensibilité de la détection, de traiter les échantillons de selles, avant de les tester en vue de détecter le ou les pathogènes associé(s) aux diarrhées et ceci, afin de retenir des composés présents dans les selles qui pourraient interférer avec leur détection.

La méthode utilisée jusqu'à ce jour consiste à réaliser une centrifugation préalablement à l'étape de détection, éventuellement en association avec un prétraitement des échantillons de selles au charbon, comme décrit par Dennehy, P. H. et al., 1994, Journal of Clinical Microbiology, 32(3) : 825-827, avec le test VIDAS® Rotavirus, dans lequel cette étape de centrifugation est obligatoire. Cette méthode, du fait de l'utilisation de centrifugeuse et de personnel de laboratoire formé, n'est donc pas adaptée pour s'adresser à un personnel hors des laboratoires d'analyses médicales, tel que médecin, infirmière, pédiatre, personnel des maisons de retraite ou des centres de gériatrie, notamment ceux utilisant des dispositifs d'immunochromatographie. De plus, même avec un personnel qualifié, la centrifugation est une étape qui génère un coût et qui est consommatrice en temps. Or, les gastro-entérites étant des phénomènes de courte durée, il est important de pouvoir agir dans les meilleurs délais pour détecter le ou les pathogène(s) à l'origine de la pathologie, d'une part, pour apporter au plus vite la réponse thérapeutique appropriée et, d'autre, part pour éviter les contaminations inter-humaines. En effet, ces pathogènes sont très facilement transmissibles, ce qui pose des problèmes de contamination dans les lieux fortement peuplés comme les maisons de retraite, les centres de gériatrie, les crèches ...

S.A. CHISHOLM : JOURNAL OF MEDICAL MICROBIOLOGY, vol. 53, no 7, 1 juillet 2004 (2004-07-01), pages 623-627, décrit l'intérêt de l'utilisation d'un test immuno-chromatographique (ImmunoCard Stat® HpSA) par détection d'antigènes pour mettre en évidence la présence d'Helicobacter pylori. Comme mentionné dans le paragraphe « Methods » en page 53, l'analyse est réalisée sur un échantillon de selles préalablement diluées et soumis à une opération de vortex.

US 2010/024530 décrit la mise en oeuvre de dispositifs dits rapides, par « lateral flow ». Il est prévu dans la partie 18, dans laquelle l'échantillon est déposé, de loger un filtre 17 permettant l'exclusion de cellules, de débris et de substances interférentes, notamment fabriqués en polymère, verre, métal poreux, ou tout autre matériau adapté pour produire un filtre de taille de pore uniforme et présentant des propriétés de filtration.

Dans US 2012/122149 le charbon actif est utilisé pour concentrer l'échantillon et nullement pour améliorer la sensibilité de la méthode. Le charbon actif est cité, mais parmi différents matériaux listés dans US 2012/122149. Mais, la détection d'antigène, n'est nullement envisagée dans US 2012/122149.

Dans ce contexte, l'invention propose un procédé et un dispositif permettant contre toute attente de simplifier la détection d'au moins un antigène d'un microorganisme, et qui soit donc adapté à la détermination de la présence dudit antigène dans les selles d'un patient, de manière à permettre de conclure, rapidement et sans appareillage coûteux et contraignant de centrifugation, si ledit patient est infecté par un pathogène responsable des gastro-entérites, tout en garantissant une sensibilité satisfaisante.

L'invention concerne un procédé de détermination de la présence d'un microorganisme cible chez un patient, à partir d'un échantillon de selles dudit patient, caractérisé en ce qu'il comprend les opérations suivantes :
- disposer d'un échantillon de selles liquides dudit patient ou d'un échantillon liquide obtenu à partir des selles dudit patient, nommé échantillon liquide,
- prétraiter l'échantillon liquide avec du charbon actif,
- détecter par immunochromatographie, également nommée immunoessai par flux latéral, en utilisant un dispositif d'immunochromatographie comprenant une zone d'application de l'échantillon, une zone de marquage et une zone réactionnelle, à partir de l'échantillon liquide prétraité obtenu, la présence éventuelle d'au moins un antigène du microorganisme cible, de manière à conclure sur la présence ou l'absence du microorganisme cible chez ledit patient.

Dans le cadre de l'invention, il a été démontré, de manière inattendue, que la mise en présence de charbon actif directement avec un échantillon liquide de selles ou avec un échantillon liquide obtenu à partir de selles d'un patient, permettait de résoudre les interférences avec des composés naturellement présents dans les échantillons, sans nécessiter contre toute attente une étape contraignante de centrifugation, favorisant ainsi l'analyse ultérieure par immunochromatographie par détection d'antigène(s) du microorganisme cible. Ceci est très important pour pouvoir bénéficier de tous les avantages des analyses par immunochromatographie, qualifiées de tests rapides, en exigeant ainsi un minimum de contraintes humaines et technologiques et rendant les analyses adaptables à toutes les conditions réelles. L'invention permet donc de détecter chez un patient une infection par un microorganisme cible dont les antigènes se trouvent, en cas d'infection dans les selles dudit patient.

Par « échantillon de selles liquides », on entend directement les selles d'un patient lorsque ces dernières sont suffisamment liquides.

Par « échantillon liquide obtenu à partir des selles », on entend soit un échantillon liquide comprenant les selles du patient et un diluant liquide, en particulier un tampon de dilution, soit un échantillon liquide obtenu à partir des selles d'un patient et d'un diluant liquide, et en particulier un tampon de dilution, après élimination des selles. Dans ce cas, les selles sont placées dans un diluant liquide, notamment un tampon de dilution, ce qui permet d'extraire des selles, les microorganismes susceptibles d'être présents, les antigènes du microorganisme cible à détecter et autres composants permettant ensuite de caractériser la présence ou non du ou des antigènes du microorganisme cible dans l'échantillon liquide obtenu, et donc par voie de conséquence de conclure sur la présence ou l'absence dudit microorganisme chez le patient dont sont originaires les selles. La quantité de selles par volume de tampon de dilution est, de préférence, de 5 à 50 mg/ml de tampon de dilution, de préférence encore de 10 à 30 mg/ml, de préférence encore de 14 à 17 mg/ml, 15 et 16 mg/ml étant préférés.

Le tampon de dilution présent dans l'échantillon liquide contient, en général, une base tampon, une protéine de charge dénaturée et un détergent. A titre d'exemple de base tampon, on peut citer un tampon phosphate, un tampon tris-HCl. A titre d'exemple de protéine de charge dénaturée, on peut citer la caséine, l'ovalbumine et l'albumine sérique bovine. A titre de détergent, on peut citer les détergents ioniques tels que le Triton™ X100 et le Tween® 20.

Le procédé selon l'invention pourra donc comprendre la préparation d'un échantillon liquide à partir de selles, par incorporation des selles dans un diluant liquide, notamment un tampon de dilution, et de préférence un tampon de dilution tel que décrit précédemment et préférentiellement avec les quantités de selles par volume de tampon de dilution précédemment mentionnées. Une élimination des selles de l'échantillon liquide, sera le plus souvent mise en oeuvre, après, avant ou lors du prétraitement de l'échantillon liquide avec le charbon actif, en fonction du mode de prétraitement mis en oeuvre.

Dans la suite, on nommera « échantillon liquide », aussi bien un échantillon de selles liquides qu'un échantillon liquide obtenu à partir des selles dudit patient. L'échantillon liquide contient au moins un antigène du micro-organisme cible qui sera à détecter.

La caractérisation de l'échantillon liquide permettant de conclure si un antigène d'un microorganisme cible est présent dans ledit échantillon liquide, se fait par la détection de la présence d'un ou plusieurs antigène(s) dudit microorganisme et permet, par voie de conséquence, de conclure si ledit antigène du microorganisme cible est ou non présent dans lesdites selles, et de ce fait de conclure si le patient, dont provient lesdites selles, est contaminé ou non par le microorganisme cible. Le microorganisme cible lui-même peut être présent ou non dans les selles et donc dans l'échantillon liquide. Cela a peu d'importance puisque l'information recherchée est de savoir si le microorganisme est présent ou non chez le patient, et pour cela la présence d'au moins un antigène du microorganisme cible est recherché.

Dans le cadre de l'invention, la détection porte sur au moins un antigène du microorganisme cible. Ledit au moins un antigène peut être une protéine sécrétée dans les selles (antigène « libre ») ou être un antigène présent dans la structure du microorganisme, notamment un antigène de surface du microorganisme. Ce dernier peut être une protéine, un sucre (polysaccharide) ou un lipopolysaccharide. Que l'on détecte un antigène libre ou un antigène dans la structure du microorganisme cible, en cas de détection positive, la conclusion sera que le patient est infecté par le microorganisme cible et, inversement, en cas de détection négative, la conclusion sera que le patient n'est pas infecté par le microorganisme cible.

Les antigènes du microorganisme cible peuvent se trouver directement accessibles pour la détection : c'est notamment le cas lorsqu'ils sont sécrétés par le microorganisme ou s'ils se trouvent en surface de ce dernier. Si le ou les antigènes ciblés ne sont pas directement accessibles pour la détection (antigène intracellulaire notamment dans le cas de bactéries), une étape préparatoire permettant de rendre les antigènes du microorganisme cible accessibles pour la détection pourra être appliquée sur l'échantillon liquide ou lors de sa préparation, selon des techniques bien connues de l'homme du métier et souvent spécifiques de chaque antigène ciblé. Cette étape préparatoire aura lieu, le plus souvent, avant le prétraitement au charbon actif, mais il est également possible de la réaliser en simultané avec le prétraitement au charbon actif. Par exemple, l'extraction des toxines A et B du *Clostridium difficile* ne nécessite guère plus qu'une dilution dans un tampon de type PBS alors que l'extraction du Galactomannan d'*Aspergillus fumigatus* nécessite une étape de chélation en présence d'EDTA suivie d'une dénaturation par chauffage à 95°C pendant 5 min. En fonction de l'antigène ciblé et de la complexité structurale du microorganisme qui le produit, l'homme du métier combinera détergents, dénaturants, agents chaotropiques et éventuellement des moyens physiques (chauffage, actions mécaniques comme le broyage), afin de détruire les structures du microorganisme et rendre l'antigène ciblé accessible pour la détection, en prenant soin de préserver sa réactivité immunologique. Dans le cas de la détection de virus, les antigènes de surface seront directement accessibles et une telle étape préparatoire sera, donc en général, non nécessaire.

Le charbon actif, également nommé charbon activé, est un charbon amorphe poreux, composé principalement d'atomes de carbone, et présentant une très grande surface spécifique qui lui confère un fort pouvoir adsorbant. De tels charbons sont généralement obtenus après une étape de carbonisation à haute température.

Dans le cadre de l'invention, le prétraitement au charbon actif permet d'adsorber les agents interférents contenus dans l'échantillon liquide, en vue d'une analyse ultérieure de l'échantillon de selles, notamment par immunochromatographie. Le prétraitement au charbon actif permet de rendre les épitopes des antigènes à détecter plus facilement accessibles aux partenaires de liaison utilisés lors de la détection. L'étape de détection dudit au moins un antigène dans le procédé selon l'invention est réalisée par immunochromatographie, également nommée immunoessal par flux latéral. Les tests du type immunoessal par flux latéral sont également nommés tests rapides, et utilisent bien souvent un dispositif sous la forme de bandelettes positionnées dans un boîtier. Ces dispositifs comprennent une zone d'application de l'échantillon, une zone de marquage et une zone réactionnelle telles que décrites plus loin. Pour plus de détails sur ces types de procédés et dispositifs pouvant être mis en oeuvre dans le cadre de l'invention, on pourra notamment se référer aux demandes WO 2004/003559, WO 2006/092103, WO 2007/081330, US 2004/0161859, WO 2012/172232 et WO 2008/018073. Ces tests rapides sont, le plus souvent, mis en oeuvre sans étape de lavage contrairement à ce qui est fait dans le cas des immunoessais sur microplaques ou utilisant le dispositif VIDAS® commercialisé par la Demanderesse. Le prétraitement au charbon actif proposé dans le cadre de l'invention a ainsi un impact plus important sur les performances diagnostiques de tels procédés et dispositifs d'immunoessai par flux latéral.

En général, le prétraitement au charbon actif comporte la mise en contact de l'échantillon liquide avec du charbon actif et la séparation du charbon actif et de l'échantillon liquide. La mise en contact de l'échantillon liquide avec du charbon actif peut être simplement réalisée, par exemple, par mélange de l'échantillon liquide avec du charbon actif ou par passage de l'échantillon liquide sur du charbon actif. Le contact devra être suffisant pour permettre au charbon actif de remplir son rôle et éliminer des agents susceptibles d'interférer dans la détection ultérieure.

Dans le cadre de l'invention, le prétraitement de l'échantillon liquide est, de préférence, réalisé avec une quantité de charbon actif de 3 à 30 g/L d'échantillon liquide, de préférence de 6 à 18 g/L, de préférence encore de 8,4 à 10,2 g/mL, une gamme de 9 à 9,6 g/mL étant préférée.

Après le prétraitement, de manière avantageuse, une séparation du charbon actif et de l'échantillon liquide est mise en oeuvre. Une telle séparation peut être effectuée, notamment, par filtration, sans nécessiter aucune étape de centrifugation. La séparation pourra intervenir simultanément avec la mise en contact, notamment lorsque le charbon actif est positionné directement sur un système de filtration sur lequel va passer l'échantillon liquide.

Le prétraitement peut être réalisé lors de l'obtention de l'échantillon liquide. Dans ce cas, le procédé selon l'invention comprend également la préparation de l'échantillon liquide et le prétraitement au charbon actif est réalisé sur l'échantillon liquide directement lors de sa préparation, par mélange des selles, d'un diluant liquide, en particulier un tampon de dilution correspondant notamment à la description précédente, et du charbon actif, suivi d'une séparation permettant de récupérer l'échantillon liquide. Le charbon actif utilisé peut être sous la forme d'une suspension dans un diluant liquide (correspondant notamment à la description précédente du tampon de dilution) et sera alors directement mélangé avec les selles, telles qu'obtenues du patient. L'échantillon liquide obtenu après la séparation ne contient donc plus de selles, ni de charbon actif. La séparation pourra être réalisée par filtration, notamment grâce à un filtre positionné dans un dispositif de prélèvement.

Le prétraitement peut également être réalisé par mélange de l'échantillon liquide, avec du charbon actif, suivi d'une séparation permettant de récupérer l'échantillon liquide. Là encore, le charbon actif incorporé à l'échantillon liquide peut être sous la forme d'une suspension dans un diluant liquide (correspondant notamment à la description précédente du tampon de dilution). L'échantillon liquide obtenu après la séparation ne contient donc plus de charbon actif. La séparation pourra être réalisée par filtration, notamment grâce à un filtre positionné dans un dispositif de prélèvement ou directement au niveau de la zone d'application du dispositif d'immunoessai, et notamment du dispositif d'immunoessai par flux latéral.

Le prétraitement peut également être réalisé par passage de l'échantillon liquide sur du charbon actif. Dans ce cas, il est possible de prévoir le passage de l'échantillon liquide sur du charbon actif positionné dans un dispositif de prélèvement ou directement sur du charbon actif positionné sur le dispositif d'immunoessai tel qu'un dispositif d'immunoessai par flux latéral, comme détaillé ci-après. Dans les deux cas, le charbon actif sera retenu et/ou immobilisé dans le dispositif (de prélèvement ou d'immunoessai) pour éviter qu'il ne soit entraîné avec l'échantillon liquide, ce qui pourrait gêner ensuite la détection/visualisation et la lecture du résultat. Il est notamment possible de réaliser un dépôt de charbon actif sur un filtre ou sur une partie du milieu de diffusion utilisé dans le dispositif d'immunoessai tel qu'un dispositif d'immunoessai par flux latéral. Un tel dépôt pourra être obtenu par imprégnation du filtre ou d'une partie du milieu de diffusion par un liquide tel que le tampon de dilution décrit précédemment contenant le charbon actif en suspension, suivi d'un séchage.

D'une manière générale, que la mise en contact de l'échantillon liquide avec le charbon actif se fasse avant le dépôt de l'échantillon liquide sur le dispositif d'immunoessai tel qu'un dispositif d'immunoessai par flux latéral, ou directement sur un tel dispositif d'immunoessai, l'échantillon liquide déposé dans le dispositif d'immunoessai, aura, de préférence, subi préalablement à son dépôt une étape d'élimination des selles. Cette étape pourra être concomitante à l'élimination du charbon actif, si la mise en contact de l'échantillon liquide avec le charbon actif est réalisée avant le dépôt de l'échantillon liquide sur le dispositif d'immunoessai.

Le charbon activé peut être sous la forme de particules de charbon activé ou de fibres de charbon activé, et en particulier, de particules ou de fibres constituées exclusivement de charbon activé, sans un quelconque enrobage. En fonction de la nature et du moment du prétraitement mis en oeuvre, les particules ou fibres de charbon activé peuvent être en suspension dans une solution, par exemple dans un tampon ou un diluant liquide, ou être incorporées dans un matériau poreux naturel ou synthétique, tel qu'un matériau du type non-tissé, à base de fibres de cellulose ou de fibres de verre. Lorsque les particules de charbon activé sont incorporées dans une partie du milieu de diffusion d'un dispositif de détection par immunochromatographie, l'organisation de la matière sera adaptée pour permettre de retenir le charbon actif et le charbon adsorbera les agents interférents et autres impuretés, tout en autorisant le passage du ou des antigènes à détecter.

A titre d'exemple, le prétraitement au charbon actif est réalisé avec du charbon actif en poudre. Selon la classification de l'ATSM (American Society for Testing and Materials), les charbons actifs en poudre sont constitués de particules dont 95 - 100% sont capables de passer à travers un tamis à mailles de taille 80 US mesh (soit environ 177 µm), ce qui correspond à une taille de particules de 80 US mesh. Le charbon actif utilisé aura, de préférence, une taille de particules de 100 à 400 US mesh, de préférence encore de 140 à 270 US mesh, et de préférence encore de 200 US mesh. A titre d'exemple, on peut citer le charbon activé Norit® CN1, dont la taille de particules appartient à la gamme 140 à 270 US mesh.

La détermination de la taille des particules de charbon actif est réalisée par tamisage selon les techniques connues de l'homme du métier. Un exemple de protocole est décrit dans la norme ASTM D2862 - 10 « Standard Test Method for Particle Size Distribution of Granular Activated Carbon ».

Le charbon actif peut également être sous la forme de fibres d'un diamètre de 2 à 50 micromètres.

Le charbon actif utilisé présentera, de préférence, une surface spécifique supérieure à 500 m²/g, de préférence supérieure à 1000 m²/g, de préférence encore supérieure à 1300 m²/g. A titre d'exemple, le charbon activé Norit® CN1 présente une surface spécifique de 1400 m²/g.

Le charbon actif sera également capable d'adsorber, de préférence, au moins 10 g de bleu de méthylène (No CAS 61-73-4) pour 100 g de charbon, de préférence au moins 20g/100g, de préférence encore au moins 25 g/100 g. A titre d'exemple, le charbon activé Norit® CN1 est capable d'adsorber 29 g/100 g.

Contrairement aux techniques antérieures, dans le procédé selon l'invention, le prétraitement avec du charbon actif n'est pas suivie d'une étape de centrifugation, ni n'inclut une étape de centrifugation.

Le procédé selon l'invention peut être utilisé pour déterminer si un microorganisme est présent ou non chez un patient, un tel microorganisme pouvant être choisi parmi les virus, bactéries et parasites, notamment du type *Clostridium difficile, Salmonella, Shigella,* rotavirus, norovirus, adénovirus, *Entamoeba histolytica* et est particulièrement adapté à la détermination de la présence d'un virus comme le rotavirus, l'adénovirus ou le norovirus qui est un virus de très petite taille.

Le genre norovirus de la famille des *caliciviridae* est un virus non enveloppé, dont la taille est comprise entre 27 et 35 nanomètres, possédant une capside icosaédrique et un génome ARN. En raison de sa diversité génétique, le genre norovirus est divisé en génogroupes, puis, en génotypes. Le séquençage de la protéine a permis de définir cinq génogroupes (I à V) dont les génogroupes I, II et IV infectant l'homme. Après analyse des séquences en acides aminés de la protéine majeure de la capside, 8 génotypes sont aujourd'hui connus pour génogroupe GI et plus de 17 génotypes pour génogroupe GII (Zheng D. et al. Norovirus classification and proposed strain nomenclature. Virology 2006 Mar 15 ; 346 :312-323. et étendus à 19 par Wang et al. Molecular epidemiology of noroviruses in children and adults with acute gastroenteritis in Wuhan China, 2007-2010. Arch. Virol. 2012 Dec ; 157 : 2417-24). La désignation adoptée par la communauté scientifique indique le génogroupe, GI ou GII, suivi du génotype GI.1, GII.4... Le procédé selon l'invention est adapté à la détermination de la présence de tous ces génotypes.

De manière dassique, la détection d'au moins un antigène du microorganisme cible dans l'échantillon liquide consistera en la détection par immunoessal de l'interaction d'au moins un antigène du microorganisme d'intérêt avec un partenaire de liaison à l'antigène tel qu'un anticorps ou fragment d'anticorps. De manière préférée, l'anticorps ou fragment d'anticorps sera spécifique de l'antigène à détecter. Il est possible d'utiliser un anticorps monoclonal ou polyclonal ou plusieurs anticorps monoclonaux. Ces anticorps seront obtenus selon les techniques bien connues de l'homme de l'art.

Bien entendu, le préfixe « immuno » dans le terme « immunoessal », par exemple, n'est pas à considérer dans la présente demande comme indiquant strictement que le partenaire de liaison est nécessairement un partenaire d'origine immunologique, tel qu'un anticorps ou un fragment d'anticorps. En effet, comme cela est bien connu de l'homme du métier, ce terme est plus largement utilisé pour désigner des tests et procédés dans lesquels le partenaire de liaison n'est pas un partenaire d'origine/de nature immunologique mais consiste, par exemple, en un récepteur de l'analyte que l'on souhaite détecter et/ou quantifier. La condition étant que le partenaire de liaison concerné soit capable de se lier à l'analyte recherché, de préférence de manière spécifique. Ainsi, il est connu de parler de test ELISA pour des tests qui utilisent des partenaires de liaison non immunologiques *stricto sensu,* appelés plus largement en anglais « ligand binding assay », que l'on pourrait traduire en langue française par « dosage utilisant la liaison à un ligand », alors que le terme « immuno » est inclus dans l'intitulé *in extenso* correspondant à l'acronyme ELISA. Dans un souci de clarté et d'uniformité, le terme « immuno » est employé dans la présente demande pour désigner toute analyse biologique utilisant au moins un partenaire de liaison adapté pour se lier à l'analyte recherché et détecter et/ou quantifier ce dernier, de préférence de manière spécifique, même quand ledit partenaire de liaison n'est pas de nature ou d'origine immunologique au sens strict.

Des exemples de partenaires de liaison n'étant pas de nature ou d'origine immunologique comprennent les nanofitines, les récepteurs à l'antigène d'intérêt, les aptamères, les DARPins ou toute autre molécule qui est connue pour avoir une interaction avec l'antigène d'intérêt.

Les nanofitines (nom commercial) sont de petites protéines qui, comme les anticorps, sont capables de se lier à une cible biologique permettant ainsi de la détecter, de la capturer ou tout simplement de la cibler au sein d'un organisme.

Les aptamères sont des oligonucléotides, généralement ARN ou ADN, identifiés dans des banques contenant jusqu'à 10¹⁵ séquences différentes, par une méthode combinatoire de sélection in vitro appelée SELEX pour «Systematic Evolution of Ligands by Exponentiel Enrichment » (Ellington AD et Szostak JW., 1990, Nature, 346 : 818-822). La plupart des aptamères sont composés d'ARN, en raison de la capacité de l'ARN à adopter des structures variées et complexes, ce qui permet de créer à sa surface des cavités de géométries variées, permettant de fixer des ligands divers. Il s'agit d'outils biochimiques d'intérêt qui peuvent être utilisés dans des applications biotechnologiques, diagnostiques ou thérapeutiques. Leur sélectivité et leurs propriétés de fixation de ligands sont comparables à celles des anticorps.

Les « DARPins » pour Designed Ankyrin Repeat ProteINS (Boersma YL et Plütckthun A, 2011, Curr. Opin. Biotechnol, 22 : 849-857) sont une autre classe de protéines permettant de mimer les anticorps et de pouvoir se fixer avec une affinité et une sélectivité élevées sur des protéines cibles. Ils dérivent de la famille des protéines ankyrines qui sont des protéines adaptatrices permettant de fixer les protéines de membrane intégrales au réseau spectrine/actine qui constitue « la colonne vertébrale » de la membrane plasmatique cellulaire. La structure des ankyrines est basée sur la répétition d'un motif d'environ 33 acides aminés et il en est de même des DARPins. Chaque motif a une structure secondaire de type hélice-coude-hélice (« helix-turn-helix »). Les DARPins contiennent au moins trois, de préférence quatre à cinq motifs répétés et sont obtenus par « screening » de banques combinatoires.

La détection dudit au moins un antigène d'intérêt par immunoessal se fera, de préférence, par une détection du type sandwich qui est une technique largement connue de l'homme du métier mettant en oeuvre deux partenaires de liaison à l'analyte. L'un des deux partenaires peut être couplé à un marqueur pour former un conjugué ou un traceur. L'autre partenaire de liaison peut être capturé sur un support solide. On parle alors de partenaire de capture pour ce dernier et partenaire de détection pour le premier.

Le signal mesuré émis lors de l'immunoessai est alors proportionnel à la quantité de l'analyte de l'échantillon biologique.

Par marqueur, on entend, notamment, toute molécule contenant un groupement réactif avec un groupement du partenaire de liaison, directement sans modification chimique, ou après modification chimique pour inclure un tel groupement, laquelle molécule est capable de générer directement ou indirectement un signal détectable. Une liste non limitative de ces marqueurs de détection directe consiste en :
- les enzymes qui produisent un signal détectable, par exemple, par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I,
- les molécules fluorescentes telles que les Alexa ou les phycocyanines, et
- les sels électrochimiluminescents tels que des dérivés organo-métalliques à base d'acridinium ou de ruthénium.

Des systèmes indirects de détection peuvent aussi être utilisés, comme, par exemple, des ligands capables de réagir avec un anti-ligand. Le ligand correspond alors au marqueur pour constituer, avec le partenaire de liaison, le conjugué.

Les couples ligand/anti-ligand sont bien connus de l'homme du métier, ce qui est le cas, par exemple, des couples suivants : biotine/streptavidine, haptène/anticorps, antigène/anticorps, peptide/anticorps, sucre/lectine, polynucléotide/complémentaire du polynudéotide.

L'anti-ligand peut alors être détectable directement par les marqueurs de détection directe décrits précédemment ou être lui-même détectable par un autre couple ligand/anti-ligand, et ainsi de suite.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR 2781802 ou WO 95/08000 de la Demanderesse.

Selon le type de marquage utilisé, l'homme du métier ajoutera des réactifs permettant la visualisation du marquage ou l'émission d'un signal détectable par tout type d'appareil de mesure approprié, comme, par exemple, un spectrophotomètre, un spectrofluorimètre, un densitomètre, un luminomètre ou encore une caméra haute définition.

La détection d'au moins un antigène est réalisée par immunochromatographie, également nommée immunoessal par flux latéral. Les dispositifs généralement utilisés dans de tels tests comprennent un milieu de diffusion qui permet la migration de l'échantillon liquide, généralement immobilisé sur un support. On distingue classiquement plusieurs zones au niveau du milieu de diffusion qui sont une zone d'application de l'échantillon liquide, une zone de marquage et une zone réactionnelle, cette dernière comprenant une zone de visualisation (également nommée zone capture) et une zone de contrôle. Ces différentes zones sont en communication de fluide. Ainsi, l'antigène à détecter, s'il est présent dans l'échantillon déposé au niveau de la zone d'application, se lie à un premier partenaire de liaison marqué au niveau de la zone de marquage, le complexe ainsi formé migre ensuite jusqu'à la zone réactionnelle, où il est immobilisé au niveau de la zone de capture par réaction avec un second partenaire de liaison lié au milieu de diffusion, et l'utilisateur peut déterminer si l'antigène est bien présent par mise en évidence d'un signal détectable, qui est déterminé par le type de marqueur associé au premier partenaire de liaison. Généralement, la présence de l'antigène dans l'échantillon est matérialisée sous la forme d'une ligne détectable, habituellement dénommée ligne test. En général, la zone réactionnelle comprend également une zone de contrôle de la migration de l'échantillon qui va indiquer à l'utilisateur que l'échantillon a migré correctement au travers du milieu de diffusion, en amont de la zone de visualisation. Ce peut être, par exemple, par la révélation d'une ligne de contrôle d'une couleur prédéterminée. On peut citer, à titre d'exemples, les demandes de brevet WO 2004/003559, WO 2006/092103, WO 2007/081330, US 2004/0161859 et WO 2012/172232 qui décrivent de tels dispositifs. Un tel dispositif est notamment commercialisé par la société bioMérieux sous la référence VIKIA® Rota-Adeno - Réf. 31111 pour détecter simultanément les rotavirus et les adénovirus.

Les procédés et dispositifs selon l'invention permettront de conclure si au moins un antigène du microorganisme d'intérêt est présent ou non dans l'échantillon liquide, et donc par voie de conséquence dans les selles du patient concerné, et ainsi de conclure si le patient est contaminé ou non par le microorganisme, et ce bien entendu, dans la limite du seuil de détection du procédé et du dispositif. Il n'est néanmoins pas exclu que le procédé selon l'invention permette un dosage quantitatif dudit au moins un antigène recherché et donc du microorganisme cible, en fonction de la technique de détection mise en oeuvre.

L'invention a également pour objet un dispositif de détection d'au moins un antigène d'un microorganisme dans un échantillon liquide comprenant :
a) un support,
b) un milieu de diffusion, fixé sur le support, qui permet la migration de l'échantillon liquide, ledit milieu de diffusion comportant :
   (i) une zone d'application de l'échantillon liquide,
   (ii) une zone de purification comprenant du charbon actif,
   (iii) une zone de marquage comprenant au moins un premier partenaire de liaison marqué, ledit premier partenaire de liaison étant capable de se lier audit au moins un antigène du microorganisme à détecter, s'il est présent dans l'échantillon liquide, formant alors un complexe premier partenaire de liaison/antigène et
   (iv) au moins une zone réactionnelle comprenant :
      - une zone de visualisation des résultats de la détection comprenant au moins un second partenaire de liaison immobilisé sur le milieu de diffusion qui est susceptible de se lier audit complexe premier partenaire de liaison/antigène,
      - une zone de contrôle de la migration qui permet de contrôler le bon fonctionnement du dispositif, située en aval de la zone de visualisation,
   lesdites zones d'application, de purification, de marquage et réactionnelle étant en communication de fluide pour permettre la diffusion du liquide.

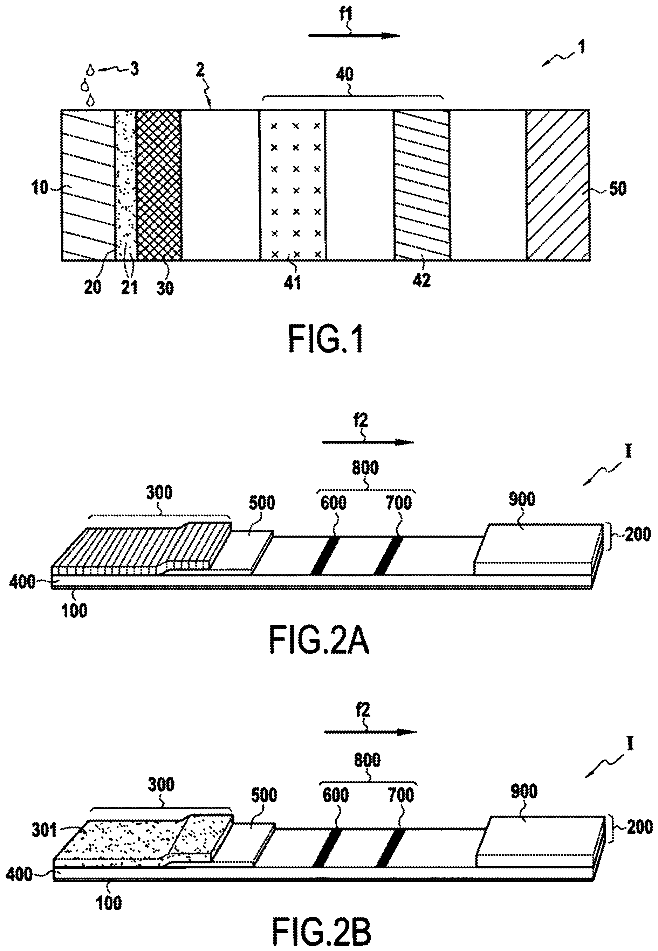

De tels dispositifs sont illustrés sur les **Figure 1** et **2B****.**
La **Figure 1** est une représentation schématique de dessus d'un exemple de dispositif de détection selon l'invention.
La **Figure 2A** est une représentation schématique en perspective d'un dispositif de détection selon l'art antérieur et la **Figure 2B** une représentation schématique en perspective d'un exemple de dispositif de détection conforme à l'invention.

Le dispositif **1** représenté **Figure 1** permet la migration de l'échantillon liquide selon le sens **f₁** et comporte un milieu de diffusion **2,** qui est fixé sur un support non représenté. Le support est, de manière connue de l'homme du métier, de nature hydrofuge, par exemple sous la forme d'une couche mince en un matériau plastique. Le support a pour fonction de rigidifier, faciliter la manipulation et protéger le milieu de diffusion **2.** Le support permet également d'imperméabiliser la face inférieure du milieu de diffusion **2** et par conséquent de canaliser le flux de l'échantillon au sein du milieu de diffusion **2.**

Le milieu de diffusion **2** comporte différentes zones successives dans le sens **f₁,** correspondant au sens de migration de l'échantillon liquide **3** qui va être déposé : la zone d'application **10** de l'échantillon liquide **3,** la zone de purification **20** comprenant du charbon actif **21,** la zone de marquage **30** comprenant au moins un premier partenaire de liaison marqué, la zone de visualisation **41** et la zone de contrôle de la migration **42** ces deux dernières constituant la zone réactionnelle **40.** Le plus souvent, à l'extrémité opposée à la zone d'application **10,** le dispositif **1** comprendra une zone d'absorption **50,** permettant de favoriser la diffusion de l'échantillon liquide **3.** La zone d'application **10** et la zone de purification **20** peuvent être confondues. Le milieu de diffusion **2** peut être constitué d'une seule couche d'une matrice poreuse ou, le plus souvent, de plusieurs couches d'une matrice poreuse, chacune des couches comprenant une ou plusieurs zones. Tout type de matériau qui est capable d'assurer le flux et le transfert d'un fluide peut être utilisé en tant que matrice poreuse. Le transfert du fluide peut être assuré par force de capillarité. Un matériau bibuleux, par exemple, du type membrane ou papier filtre, qui absorbe facilement un liquide et au travers duquel le liquide est transporté par capillarité, peut être utilisé. En général, un tel dispositif est placé dans une cassette ou boîtier, non représenté, comprenant un puits pour le dépôt de l'échantillon au niveau de la zone d'application et une fenêtre permettant de visualiser la zone réactionnelle.

La zone d'application (et de purification) et/ou la zone de marquage et/ou la zone d'absorption notamment peuvent être constituées par des couches poreuses rapportées sur, ou chevauchant partiellement, une première couche déposée sur toute la surface du support, ou le plus souvent sur une première couche déposée sur une partie seulement de la surface du support. Cette première couche déposée sur le support sert de membrane analytique et incorpore la zone réactionnelle (zone de visualisation et zone de contrôle de la migration).

Ainsi, de manière à faciliter la fabrication du dispositif et favoriser la diffusion de l'échantillon liquide déposé (selon le sens f2 en **Figure 2**), les différentes zones du milieu de diffusion seront constituées de plusieurs couches d'une matrice poreuse, venant se chevaucher et étant en communication de fluide, comme illustrée sur la **Figure 2B****,** détaillée en relation avec les exemples. Il est possible notamment d'utiliser différentes couches de membranes ou papier filtre. En particulier, comme illustré sur la **Figure 2B****,** une couche correspondant à la zone de purification **300,** et également à la zone d'application, comprenant du charbon actif **301** pourra chevaucher partiellement une couche correspondant à la zone de marquage **500.** La couche correspondant à zone de marquage **500** pourra, quant à elle, chevaucher la membrane analytique **400** incorporant les zones de visualisation **600** et de contrôle de la migration **700.** La membrane analytique **400** pourra s'étendre sur toute la surface du support **100,** comme illustré sur la **Figure 2B****,** ou seulement sur une partie du support. De tels chevauchements sont classiquement utilisés dans l'état de la technique (voir notamment WO 2012/172232 et WO 2008/018073) et permettent notamment d'assurer la continuité du flux de l'échantillon liquide.

La différence entre le dispositif de l'invention illustré sur la **Figure 2B** et le support de l'art antérieur illustré sur la **Figure 2A** est la présence de charbon actif **301** dans la zone **300.**

De manière classique, dans le dispositif selon l'invention, le premier et le deuxième partenaires de liaison pourront être tels que décrits précédemment, et notamment des anticorps ou fragments d'anticorps, et/ou le milieu de diffusion pourra être un matériau fibreux, notamment en fibres cellulosiques ou en fibres de verre. Le plus souvent, le matériau fibreux est en nitrocellulose. Il est, par exemple, possible d'utiliser pour la membrane analytique un milieu fibreux en nitrocellulose directement solidaire d'un support, du type polyester notamment.

La visualisation des réactions immunologiques, c'est-à-dire de la liaison antigène/partenaires de liaison, peut être effectuée par tout moyen de détection par l'intermédiaire d'un marquage du premier partenaire de liaison avec un marqueur. Ainsi, le premier partenaire de liaison peut être lié à des particules marquées, capables de générer un signal détectable, c'est-à-dire comprenant un marqueur, sous la forme d'un composé ou d'une substance qui peut être détecté par des moyens visuels, fluorescents, instrumentaux. Une liste non limitative de ces marqueurs sont les particules métalliques ou d'un alliage, telles que les particules d'or colloïdales, les particules de polymère, telles que les particules de latex colorés, les particules magnétiques, les molécules fluorescentes, les molécules chimioluminescentes ... Le signal généré au niveau de la zone de visualisation des résultats et le signal généré au niveau de la zone contrôle de la migration, qui correspondent à un résultat positif, pourront être de nature identique ou différente. Par exemple, dans le cadre de l'utilisation de latex colorés, ils pourront être d'une même couleur ou de couleur différente.

Selon un mode de réalisation préféré, le procédé de détection selon l'invention met en oeuvre un dispositif décrit dans la présente demande de brevet.

Les procédés et dispositifs selon l'invention de détermination de la présence d'un microorganisme chez un patient sont compatibles avec des situations d'urgence. Les symptômes d'une gastro-entérite d'origine virale ne durant que quelques jours, le procédé et le dispositif selon l'invention, utilisés en première intention, permettront de prendre rapidement les mesures nécessaires pour protéger rapidement le patient et son environnement. L'interprétation du résultat est possible 10 minutes après le dépôt de l'échantillon dans la zone d'application. Dans les établissements de soins, la mise en évidence du norovirus par un test immunochromatographique utilisant le procédé ou le dispositif selon l'invention conduira à renforcer les mesures d'hygiène c'est-à-dire isoler le malade et accentuer la désinfection des surfaces pour éviter une épidémie. Par ailleurs, un résultat positif dans le cadre de la détection d'un virus, permettra d'écarter une infection bactérienne qui pourrait conduire à une hospitalisation chez les personnes âgées et de limiter l'usage d'antibiotiques inutile dans le cas d'une infection virale.

Les exemples ci-après permettent d'illustrer l'invention, mais n'ont aucun caractère limitatif.

### Exemple 1. Préparation d'un dispositif d'immunochromatographie pour la détection du norovirus comprenant une zone de purification à base de charbon actif.

### Préparation du dispositif

### Préparation des « Sample Pads »

Le « Sample Pad » est une bande de fibre de verre de dimensions 8 cm x 1,7 cm découpée à partir d'une membrane obtenue auprès de la société Alhstrom (Cat. No. Grade 8975, Helsinki, Finlande). Avant l'assemblage des bandelettes d'immunochromatographie, le « Sample Pad » est classiquement plongé dans un bain dans un tampon de saturation.

Dans le dispositif d'immunochromatographie de contrôle (Dispositif REF), le « Sample Pad » est plongé pendant 4h dans un bain avec un tampon contenant des sucres et de la caséine. Il est ensuite séché pendant 12 à 18 heures à 37°C.

Dans le dispositif d'immunochromatographie de l'invention (Dispositif CHARBON), le « Sample Pad » subit, dans un premier temps, le même traitement que le dispositif d'immunochromatographie de contrôle. Ensuite, il est plongé dans un bain avec un réactif à base de charbon actif. Ce réactif à base de charbon contient 0,65 g/L de base Tris, 6,83 g/L de Tris-HCl, 8,55 g/L de NaCl, 0,05% de Tween® 20, 1% d'albumine sérique bovine et 10 g/L de charbon actif (Norit CN1, Norit Nederland BV, Amerstoort, Hollande). Le pH est ajusté à 7,2 avant l'ajout du charbon actif. Après 1 heure, le « Sample Pad » est enlevé du bain, déposé sur une grille et séché à l'étuve 1h à 37°C.

### Préparation du « Conjugate Pad », zone de marquage

Des particules de latex rouges PL-Latex Carbonyl HiDyenRed 400 nm commercialisées par la société Agilent Technologies (Cat. No. PL6104-614#) sont coatées par adsorption par un mélange de deux anticorps polyclonaux IgG de lapin dirigés contre le norovirus, les anticorps 542 et 544 (bioMérieux). Après incubation pendant 12 à 18 heures à température ambiante, les particules sont saturées par un tampon à base de caséine afin d'éviter les adsorptions non spécifiques. Ces particules sont ensuite réparties sur un support en fibre de verre (Cat. No. Grade 8975, Alhstrom Helsinki, Finlande), qui est séché une nuit à 37°C.

### Préparation de la membrane analytique, zone réactionnelle

Trois anticorps monoclonaux de souris dirigés contre des antigènes du norovirus, les dones 1H3C3, 11H12 et 2A7 (bioMérieux) sont mélangés et dilués en tampon phosphate salin (PBS). La solution ainsi préparée est répartie, en utilisant un appareil BIODOT (nom commercial), sur une membrane de nitrocellulose supportée par un renfort en polyester (Unisart® CN 140 backed, Sartorius, Cat. No. 1UN14ER050020), constituant ainsi la zone de visualisation, nommée ligne de test (T). Un anticorps polyclonal dirigé contre les immunoglobulines G de lapin est dilué dans un tampon PBS. Cette solution est répartie dans la zone de contrôle de la migration, située en aval de la zone de visualisation, nommée ligne de contrôle (C). La membrane est ensuite séchée à l'étuve pendant 12/18 heures à 37°C, puis, conservée en poche aluminium scellée avec un sachet déshydratant afin de la préserver de l'humidité.

### Préparation des cassettes

Les bandelettes sont fabriquées en déposant sur la membrane analytique de nitrocellulose supportée ainsi formée : un « Sample Pad » jouant le rôle de zone d'application de l'échantillon et de zone de purification (membrane en fibre de verre qui sert de filtre pour l'échantillon avant contact avec les particules), le « Conjugate Pad » (zone de marquage) et un « Absorbant Pad » (un absorbant qui a la capacité d'adsorber le reste l'échantillon après migration le long des différents types de « Pad », jouant le rôle de zone d'absorption). Ce dernier a été fabriqué à partir du papier filtre pour huile (gamme « automotive filter paper ») de la société Hangzhou Xinhua Paper Industry Co. Ltd (Hangzhou Tonglu, Chine). Cet assemblage est ensuite découpé en bandelettes de 4 mm de large.

Le dispositif d'immunochromatographie de l'invention (Dispositif CHARBON) est présenté **Figure 2B** et ne diffère du dispositif présenté sur la **Figure 2A** (Dispositif REF) que par le fait que la première zone **300** jouant à la fois le rôle de zone d'application de l'échantillon et de zone de purification, comprend du charbon actif **301.** Ces dispositifs permettent la migration de l'échantillon liquide selon le sens **f₂** et comportent un milieu de diffusion multicouche **200.** Le milieu de diffusion multicouche **200** comprend une membrane analytique **400** avec support intégré **100** qui forme la première couche et comporte différentes zones successives dans le sens **f₂** : une première zone **300** d'application et de purification de l'échantillon, comprenant le charbon actif **301,** une zone de marquage **500** comprenant au moins un premier partenaire de liaison marqué, une zone de visualisation **600** et une zone de contrôle de la migration **700,** ces deux dernières constituant la zone réactionnelle **800.** Par endroit, le milieu de diffusion est multicouche. On notera que la couche correspondant à la zone **300** d'application sur laquelle se fait le dépôt de l'échantillon se chevauche avec une couche qui comporte la zone de marquage **500.** La couche comportant la zone de marquage **500** se chevauche ensuite avec la couche nommée membrane analytique **400,** qui comporte la zone de visualisation **600** et la zone de contrôle de la migration **700,** pour assurer la continuité du flux et favoriser la diffusion du liquide par capillarité. A l'extrémité opposée à la zone **300,** le dispositif comprend une zone d'absorption **900,** positionnée sur la couche **400** et qui permet de favoriser la diffusion de l'échantillon.

Puis, ces bandelettes sont scellées individuellement dans des cassettes en plastique comprenant un puits échantillon et une fenêtre de visualisation, prêtes à l'emploi.

### Mode opératoire du test immunochromatographique.

- retirer la cassette de son sachet et la placer sur une surface propre et plane,
- déposer dans le puits échantillon 75µL de l'échantillon à tester,
- déclencher le chronomètre puis lire le test 10 minutes après le dépôt.

Le statut des échantillons est déterminé en fonction ou non de la présence d'une ligne colorée d'intensité variable sur la membrane de nitrocellulose. L'intensité de la ligne colorée varie de L1 (absence de ligne rouge) à L10 (présence d'une ligne rouge intense). La comparaison avec une carte de lecture permet d'objectiver l'évaluation de l'intensité de la couleur de la ligne colorée.

L'interprétation des résultats se fait selon le tableau 1 suivant :

**Tableau 1**

| Lecture | Résultats | Interprétation |
|---|---|---|
| Ligne d'intensité < L4 | Négatif | Absence de l'analyte recherché |
| Ligne d'intensité = L4 ou L5 | Positif, mais proche de la limite de détection du kit | Présence a priori de l'analyte recherché |
| Ligne d'intensité > L5 | Positif | Présence de l'analyte recherché |

### Détection du norovirus par immunochromatographie

Les selles sont diluées individuellement dans le diluant VIKIA® Rota/Adeno (Cat. No. 31111, bioMérieux) à raison de 25 mg de selles pour 1500 µL de diluant puis vortexées 15 sec et déposées à raison de 75 µL dans le puits de la cassette. Les résultats sont donnés dans le Tableau 2 suivant.

**Tableau 2**

| | Selles positives pour norovirus* | |
|---|---|---|
| Code Echantillon | 87 | 437 |
| Dispositif REF | L3 | L5 |
| Dispositif CHARBON | L4 | L6 |

| | | |
|---|---|---|
| * : selles de patients infectés par le norovirus | | |

L'utilisation du dispositif d'immunochromatographie de l'invention, comprenant une zone de purification intégrant du charbon actif, permet d'améliorer la détection des antigènes dans les deux échantillons sélectionnés ci-dessus.

### Exemple 2. Utilisation d'un dispositif de prélèvement avec filtre lors du prétraitement des échantillons de selles puis détection du norovirus par immunochromatographie.

### Préparation du dispositif de prélèvement des selles avec filtre

Ce prototype est réalisé à partir du tube de prélèvement (tube à bouchon vert) du kit VIKIA® Rota/Adeno (Cat. No. 31111, bioMérieux). La tige de prélèvement blanche contenue dans le bouchon vert est enlevée, un disque Whatman GF/B (filtre 1 µm) de diamètre 6 mm (Cat. No. 1821110) est installé au fond du bouchon, puis l'extrémité de la tige (opposée au prélèvement) est replacée dans le bouchon afin de maintenir le filtre en place. L'objectif de ce filtre est de retenir les particules de charbon et d'obtenir un échantillon pouvant être déposé directement dans le puits de la cassette.

### Prétraitement des selles et immunochromatographie

Trois méthodes de prétraitement des selles ont été comparées :

### CAS 1 : REF

Les selles sont diluées individuellement dans le diluant VIKIA® Rota/Adeno (Cat. No. 31111, bioMérieux) à raison de 50 mg de selles pour 3000 µL de diluant. Les selles sont vortexées 15 secondes. L'échantillon ainsi obtenu est déposé à raison de 75 µL dans le puits de la cassette d'immunochromatographie.

### Cas 2 : CENTRIFUGATION (traitement au charbon + centrifugation) et Cas 3 : INVENTION

Le même réactif à base de charbon actif que l'Exemple 1 est utilisé. Les selles sont diluées individuellement dans le réactif charbon à raison de 50 mg de selles pour 3000 µL de diluant puis :
- CAS 2 : sont vortexées 15 secondes puis centrifugées 5 min à 12000 g.
- CAS 3 : sont réparties dans le dispositif de prélèvement avec filtre préparé précédemment.

Dans chaque cas, après avoir cassé la tétine présente à l'extrémité du bouchon, 75 µL de filtrat sont déposés dans le puits de la cassette.

Le tableau 3 ci-dessous reprend les conditions.

**Tableau 3**

| CAS 1 | | CAS 2 | | CAS 3 | |
|---|---|---|---|---|---|
| Selles diluées diluant VIKIA® Rota/Adéno (REF) | | Selles diluées réactif charbon | | | |
| Diluant VIKIA® Rota/Adéno | 3000 µL | Réactif charbon | 3000 µL | | |
| Selles | 50 mg | Selles | 50 mg | | |
| Vortex | 15 secondes | Vortex | 15 secondes | - | - |
| - | - | Centrifugation | 5 min 12000 G | Filtration | Préparation répartie dans le prototype |
| Dépôt | 75 µL | Dépôt | 75 µL | Dépôt | 75 µL |

Pour chaque cas, le volume de dépôt est de 75 µL et la lecture est réalisée 10 minutes après le dépôt de l'échantillon. Les tests sont réalisés avec un dispositif REF comme décrit à l'Exemple 1, et lus et interprétés de la même manière que dans l'Exemple 1. Les résultats sont donnés dans le tableau 4 ci-après.

**Tableau 4**

| | | CAS 1 REF Selles diluées diluant VIKIA® Rota/Adéno | CAS 2 CENTRIFUGATION Selles diluées réactif charbon + centrifugation | CAS 3 INVENTION Selles diluées réactif charbon + filtration |
|---|---|---|---|---|
| | Bouchon | Référence | Référence | Filtre 1µm Diamètre 0,6 cm |
| Echantillon contrôle | Diluant VIKIA® Rota/Adeno | L1 | L1 | L1 |
| Echantillons positifs pour norovirus * | E8909 GII.3 | L5 | L8 | L7-8 |
| | E9982 GII.4 | L4 | L9 | L6 |
| | E9918 GI.4 | L4 | L7 | L6-7 |
| | E8624 GI | L8 | L9 | L9 |

| | | | | |
|---|---|---|---|---|
| * : selles de patients infectés par le norovirus | | | | |

- L'utilisation du charbon ne dégrade pas la spécificité.
- Les échantillons proches de la limite de détection (L4/L5) avec la condition référence (CAS 1) sont très positifs avec le traitement au charbon (CAS 2 et 3).
- L'intensité de la bande de lecture est plus élevée sur les échantillons traités au charbon.
- Pour les échantillons positifs, avec un traitement au charbon, la ligne de test apparaît plus rapidement et la membrane est plus propre (les selles ne colorent plus la membrane).
- Les résultats obtenus en traitant les échantillons avec un diluant à base de charbon confirment le gain de sensibilité.
- Contre toute attente, l'utilisation de charbon actif, sans étape de centrifugation, permet d'obtenir des résultats dont l'interprétation clinique est identique aux résultats obtenus avec une étape de centrifugation. Dans les deux cas, les résultats sont positifs (L ≥ 6), alors qu'avec la référence, les résultats sont positifs *a priori.* Une séparation par filtration plutôt que par centrifugation permet de conserver une sensibilité satisfaisante.

## Revendications

1. Procédé de détermination de la présence d'un microorganisme cible chez un patient, à partir d'un échantillon de selles dudit patient, **caractérisé en ce qu'**il comprend les opérations suivantes :
- disposer d'un échantillon de selles liquides dudit patient ou d'un échantillon liquide obtenu à partir des selles dudit patient, nommé échantillon liquide,
- prétraiter l'échantillon liquide avec du charbon actif,
- détecter par immunochromatographie, également nommée immunoessal par flux latéral, en utilisant un dispositif d'immunochromatographie comprenant une zone d'application de l'échantillon, une zone de marquage et une zone réactionnelle, à partir de l'échantillon liquide prétraité obtenu, la présence éventuelle d'au moins un antigène du microorganisme cible, de manière à conclure sur la présence ou l'absence du microorganisme cible chez ledit patient.

2. Procédé de détermination selon la revendication 1 **caractérisé en ce que** le prétraitement avec du charbon actif comporte la mise en contact de l'échantillon liquide avec du charbon actif et la séparation de l'échantillon liquide ainsi obtenu et du charbon actif.

3. Procédé de détermination selon la revendication 1 ou 2 **caractérisé en ce qu'**il comprend la préparation de l'échantillon liquide de selles à partir desdites selles dudit patient et **en ce que** le prétraitement avec du charbon actif est réalisé lors de la préparation de l'échantillon liquide, par mélange des selles, d'un diluant liquide et du charbon actif, suivi d'une séparation permettant de récupérer l'échantillon liquide, sans selles ni charbon actif.

4. Procédé de détermination selon la revendication 1 ou 2 **caractérisé en ce que** le prétraitement est réalisé par mélange de l'échantillon liquide, avec du charbon actif, suivi d'une séparation permettant de récupérer l'échantillon liquide sans charbon actif.

5. Procédé de détermination selon l'une des revendications 1 à 4 **caractérisé en ce que** la séparation est réalisée par filtration.

6. Procédé de détermination selon l'une quelconque des revendications 1 à 5 **caractérisé en ce que** le prétraitement est réalisé par passage de l'échantillon liquide sur du charbon actif positionné dans un dispositif de prélèvement de l'échantillon ou dans le dispositif d'immunochromatographie.

7. Procédé de détermination selon l'une des revendications 1 à 6 **caractérisé en ce que** l'échantillon liquide contient un diluant comprenant un tampon, une protéine de charge dénaturée et un détergent.

8. Procédé de détermination selon l'une quelconque des revendications 1 à 7 **caractérisé en ce que** le microorganisme est choisi parmi les virus, bactéries et parasites et est, de préférence, un rotavirus, un adénovirus, ou, préférentiellement, un norovirus.

9. Procédé de détermination selon l'une quelconque des revendications 1 à 8 **caractérisé en ce que** la détection consiste en la détection de l'interaction d'au moins un antigène du microorganisme d'intérêt avec au moins un partenaire de liaison audit au moins un antigène, ledit partenaire de liaison étant, de préférence, un anticorps ou fragment d'anticorps.

10. Dispositif **(I, 1**) de détection d'au moins un antigène d'un microorganisme cible dans un échantillon liquide (**3**) comprenant :
a) un support **(100),**
b) un milieu de diffusion poreux (**2, 200),** fixée sur le support **(100),** qui permet la migration de l'échantillon liquide **(3),** ledit milieu de diffusion (**2, 200**) comportant :
(i) une zone d'application (**10, 300**) de l'échantillon liquide **(3),**
(ii) une zone de purification (**20, 300**) comprenant du charbon actif **(21, 301),**
(iii) une zone de marquage (**30, 500**) comprenant au moins un premier partenaire de liaison marqué, ledit premier partenaire de liaison étant capable de se lier audit au moins un antigène du microorganisme à détecter, s'il est présent dans l'échantillon liquide, formant alors un complexe premier partenaire de liaison/antigène, et
(iv) au moins une zone réactionnelle (**40, 800**) comprenant :
une zone de visualisation **(41, 600)** des résultats de la détection comprenant au moins un second partenaire de liaison, immobilisé sur le milieu de diffusion qui est susceptible de se lier audit complexe premier partenaire de liaison/antigène,
une zone de contrôle de la migration (**42, 700**) qui permet de contrôler le bon fonctionnement du dispositif, située en aval de la zone de visualisation (**41, 600),**
lesdites zones d'application (**10, 300),** de purification (**20, 300**), de marquage **(30, 500**) et réactionnelle **(40, 800**) étant en communication pour permettre la diffusion du liquide.

11. Dispositif **(I, 1**) selon la revendication 10 **caractérisé en ce que** le premier et le deuxième partenaires de liaison sont des anticorps ou fragments d'anticorps et/ou le milieu de diffusion est un matériau fibreux, notamment en fibres cellulosiques ou en fibres de verre.

12. Procédé de détection selon l'une quelconque des revendications 1 à 9 **caractérisé en ce qu'**il met en oeuvre un dispositif **(I)** selon la revendication 10 ou 11.

## Patentansprüche

1. Verfahren zum Bestimmen der Anwesenheit eines Zielmikroorganismus bei einem Patienten aus einer Stuhlprobe des Patienten, **dadurch gekennzeichnet, dass** es die folgenden Arbeitsgänge aufweist:
- Erhalten einer flüssigen Stuhlprobe des Patienten oder einer flüssigen Probe, die aus dem Stuhl des Patienten erhalten wird, die flüssige Probe genannt wird,
- Vorbehandeln der flüssigen Probe mit Aktivkohle,
- durch Immunochromatographie, auch Lateral-Flow-Immunoassay genannt, unter Verwendung von einer immunochromatographischen Vorrichtung, umfassend eine Probenaufbringungszone, eine Markierungszone und eine Reaktionszone, Erfassen aus der erhaltenen vorbehandeln flüssigen Probe die eventuelle Anwesenheit mindestens eines Antigens des Zielmikroorganismus, um auf die Anwesenheit oder Abwesenheit des Zielmikroorganismus bei dem Patienten zu schließen.

2. Verfahren zum Bestimmen nach Anspruch 1, **dadurch gekennzeichnet, dass** das Vorbehandeln mit Aktivkohle das Inkontaktbringen der flüssigen Probe mit Aktivkohle und das Trennen der auf diese Weise erhaltenen flüssigen Probe und der Aktivkohle aufweist.

3. Verfahren zum Bestimmen nach Anspruch 1 oder 2, **dadurch gekennzeichnet, dass** es das Vorbereiten der flüssigen Stuhlprobe aus dem Stuhl des Patienten aufweist und dadurch, dass das Vorbehandeln mit Aktivkohle beim Vorbereiten der flüssigen Probe durch Mischen des Stuhls, eines flüssigen Verdünnungsmittels und der Aktivkohle durchgeführt wird, worauf ein Trennen folgt, das ermöglicht, die flüssige Probe ohne Stuhl oder Aktivkohle zurückgewinnen.

4. Verfahren zum Bestimmen nach Anspruch 1 oder 2, **dadurch gekennzeichnet, dass** das Vorbehandeln durch Mischen der flüssigen Probe mit Aktivkohle durchgeführt wird, worauf ein Trennen folgt, das ermöglicht, die flüssige Probe ohne Aktivkohle zurückzugewinnen.

5. Verfahren nach einem der Ansprüche 1 bis 4, **dadurch gekennzeichnet, dass** das Trennen durch Filtern durchgeführt wird.

6. Verfahren zum Bestimmen nach einem der Ansprüche 1 bis 5, **dadurch gekennzeichnet, dass** das Vorbehandeln durch Leiten der flüssigen Probe über Aktivkohle durchgeführt wird, die in einer Vorrichtung zur Entnahme der Probe oder in der immunochromatographischen Vorrichtung angeordnet wird.

7. Verfahren zum Bestimmen nach einem der Ansprüche 1 bis 6, **dadurch gekennzeichnet, dass** die flüssige Probe ein Verdünnungsmittel enthält, das einen Puffer, ein denaturiertes Ladeprotein und ein Detergens umfasst.

8. Verfahren zum Bestimmen nach einem der Ansprüche 1 bis 7, **dadurch gekennzeichnet, dass** der Mikroorganismus aus Viren, Bakterien und Parasiten ausgewählt wird und vorzugsweise ein Rotavirus, ein Adenovirus oder vorzugsweise ein Norovirus ist.

9. Verfahren zum Bestimmen nach einem der Ansprüche 1 bis 8, **dadurch gekennzeichnet, dass** das Nachweisen in dem Nachweisen der Wechselwirkung mindestens eines Antigens des Mikroorganismus von Interesse mit mindestens einem Bindungspartner an das mindestens eine Antigens besteht, wobei der Bindungspartner vorzugsweise ein Antikörper oder ein Antikörperfragment ist.

10. Vorrichtung (I, 1) zum Nachweisen von mindestens einem Antigen eines Zielmikroorganismus in einer flüssigen Probe (3), umfassend:
a) einen Träger (100),
b) ein poröses Diffusionsmedium (2, 200), das auf dem Träger (100) befestigt ist, das die Migration der flüssigen Probe (3) ermöglicht, wobei das Diffusionsmedium (2, 200) umfasst:
(i) eine Aufbringungszone (10, 300) der flüssigen Probe (3),
(ii) eine Reinigungszone (20, 300), umfassend Aktivkohle (21, 301),
(iii) eine Markierungszone (30, 500), die mindestens einen ersten markierten Bindungspartner umfasst, wobei der erste Bindungspartner geeignet ist, an das mindestens eine Antigen des nachzuweisenden Mikroorganismus zu binden, falls er in der flüssigen Probe vorhanden ist, wodurch dann ein erster Bindungspartner/Antigen-Komplex gebildet wird, und
(iv) mindestens eine Reaktionszone (40, 800), umfassend:
einen Anzeigebereich (41, 600) der Nachweisergebnisse, umfassend mindestens einen zweiten Bindungspartner, der auf dem Diffusionsmedium immobilisiert ist, der geeignet ist, sich an den ersten Bindungspartner/Antigen-Komplex zu binden,
eine Kontrollzone der Migration (42, 700), die ermöglicht, das ordnungsgemäße Funktionieren der Vorrichtung zu überprüfen, die vorgelagert vor dem Anzeigebereich (41, 600) angeordnet ist, wobei die Aufbringungs- (10, 300), die Reinigungs- (20, 300), die Markierungs- (30, 500) und die Reaktionszone (40, 800) miteinander in Verbindung stehen, um die Diffusion der Flüssigkeit zu ermöglichen.

11. Vorrichtung (I, 1) nach Anspruch 10, **dadurch gekennzeichnet, dass** der erste und der zweite Bindungspartner Antikörper oder Antikörperfragmente sind und/oder das Diffusionsmedium ein Fasermaterial, insbesondere aus Cellulosefasern oder aus Glasfasern, ist.

12. Nachweisverfahren nach einem der Ansprüche 1 bis 9, **dadurch gekennzeichnet, dass** es eine Vorrichtung (I) nach Anspruch 10 oder 11 einsetzt.

## Claims

1. A determination method for determining the presence of a target microorganism in a patient from a sample of said patient's stools, the method being **characterized in that** it comprises the following operations:
- obtaining a sample of liquid stools of said patient or a liquid sample obtained from stools of said patient, referred to as the liquid sample;
- pretreating the liquid sample with activated carbon; and
- using immunochromatography, also known as lateral flow immunoassay, with an immunochromatography device having a sample application zone, a marking zone, and a reaction zone, to detect from the resulting pretreated liquid sample the possible presence of at least one antigen of the target microorganism so as to come to a conclusion about the presence or the absence of the target microorganism in said patient.

2. A determination method according to claim 1, **characterized in that** the pretreatment with activated carbon includes putting the liquid sample into contact with the activated carbon and separating the liquid sample as obtained thereby from the activated carbon.

3. A determination method according to claim 1 or claim 2, **characterized in that** it includes preparing the liquid sample of stools from said stools of said patient, and **in that** the pretreatment with the activated carbon is performed while preparing the liquid sample by mixing together stools, a liquid diluant, and activated carbon, followed by separating, enabling the liquid sample to be recovered without stools and without the activated carbon.

4. A determination method according to claim 1 or claim 2, **characterized in that** the pretreatment is performed by mixing the liquid sample with activated carbon followed by separating, enabling the liquid sample to be recovered without the activated carbon.

5. A determination method according to any one of claims 1 to 4, **characterized in that** separating is performed by filtering.

6. A determination method according to any one of claims 1 to 5, **characterized in that** the pretreatment is performed by passing the liquid sample over activated carbon positioned in a sampler device for taking the sample or in the immunochromatography device.

7. A determination method according to any one of claims 1 to 6, **characterized in that** the liquid sample contains a diluant comprising a buffer, a denatured charge protein, and a detergent.

8. A determination method according to any one of claims 1 to 7, **characterized in that** the microorganism is selected from viruses, bacteria, and parasites, and is preferably a rotavirus, an adenovirus, or more preferably a norovirus.

9. A determination method according to any one of claims 1 to 8, **characterized in that** detection consists in detecting the interaction of at least one antigen for the microorganism of interest with at least one binding partner for binding to said at least one antigen, said binding partner preferably being an antibody or an antibody fragment.

10. A device (I, 1) for detecting at least one antigen of a target microorganism in a liquid sample (3), the device comprising:
a) a support (100); and
b) a porous diffusion medium (2, 200) fixed on the support (100), and enabling the liquid sample (3) to migrate, said diffusion medium (2, 200) comprising:
i) an application zone (10, 300) for applying the liquid sample (3);
ii) a purification zone (20, 300) including activated carbon (21, 301);
iii) a marking zone (30, 500) including at least a first marked binding partner, said first binding partner being capable of binding with said at least one antigen of the microorganism that is to be detected, if present in the liquid sample, then forming a first binding partner and antigen complex; and
iv) at least one reaction zone (40, 800) comprising:
- a display zone (41, 600) for displaying the results of the detection and comprising at least one second binding partner held stationary on the diffusion medium and suitable for binding with said first binding partner and antigen complex;
- a migration verification zone (42, 700) enabling proper operation of the device to be verified, which zone is situated downstream from the display zone (41, 600); and
- said application zone (10, 300), purification zone (20, 300), marking zone (30, 500), and reaction zone (40, 800) being in communication to enable the liquid to diffuse.

11. A device (I, 1) according to claim 10, **characterized in that** the first and second binding partners are antibodies or antibody fragments and/or the diffusion medium is a fiber material, in particular made of cellulose fibers or of glass fibers.

12. A detection method according to any one of claims 1 to 9, **characterized in that** it uses a device (I) according to claim 10 or claim 11.
